# EUROPEAN PATENT APPLICATION

(11) **EP 0 641 858 A1**
(43) Date of publication of application: **08.03.1995**
(21) Application number: 92906236.2
(22) Date of filing: 03.03.1992
(51) Int. Cl.: C12N 9/64, A61K 37/54

(54) **SERUM CALCIUM DEPRESSING FACTOR**

(30) Priority: 06.03.1991 JP 40073/91
(71) Applicant: Chugai Seiyaku Kabushiki Kaisha, Tokyo 115 (JP)
(72) Inventor: TOMOMURA, Akito, Kagoshima-shi, Kagoshima 891-01 (JP); SAHEKI, Takeyori, Kagoshima-shi, Kagoshima 891-01 (JP); TAKAOKA, Yoshito, Toshima-ku, Tokyo 171 (JP)
(74) Representative: Davies, Jonathan Mark
(86) International application number: JP9200251
(87) International publication number: WO9215615

(57) **Abstract**

A novel serum calcium depressing factor derived from swine pancreas, which has a molecular weight of 26,500 to 28,000 D as determined by polyacrylamide gel electrophoresis, an isoelectric point of about 4.5 as determined by isoelectric electrophoresis, and an amino acid sequence of Val-Val-Gly-Gly-Gln-Asn-Ala-Ile-Pro-His-Ser-Trp-Pro-Trp-Gln-Ile-Arg-Leu- at or near the N-terminus. It has a protease activity and is inhibited by PMSF, chymostatin and trypsin inhibitor but not by APMSF and E-64. The novel depressing factor is expected to be applicable as a medicine for treating and preventing bone diseases such as osteoporosis.

## Description

### TECHNICAL FIELD

The present invention relates to a serum calcium decreasing factor of pig origin, a process for the production thereof and a pharmaceutical composition containing said factor.

### BACKGROUND ART

Although the presence of a serum calcium decreasing factor is suggested by Takaoka et al. [Nippon Iji Sinpo 2951, 15-20 (1980)], it has not yet been purified, and its properties have not been clarified.

Accordingly, the present invention is intended to provide a novel isolated serum calcium decreasing factor of pig origin.

### DISCLOSURE OF THE INVENTION

The present inventors succeeded in isolating and purifying the aimed serum calcium decreasing factor from porcine pancreas, clarified the characteristics of the substance, and clarified some of the biological properties thereof, and thus accomplished the present invention. Namely, the present invention provides a novel serum calcium decreasing factor of pig origin having the following properties:
(1) decreasing the serum calcium level in a dose-dependent manner in mice;
(2) having a molecular weight of about 26,500 to 28,000D, as estimated by SDS-polyacrylamide gel electrophoresis;
(3) having an isoelectric point of about 4.5 as estimated by isoelectric focusing;
(4) having the following amino acid sequence (SQ IN No. 1) at the N-terminus or near to the N-terminus: Val-Val-Gly-Gly-Gln-Asn-Ala-Ile-Pro-His-Ser-Trp-Pro-Trp-Gln-Ile-Arg-Leu-;
(5) having protease activity, and being inhibited by protease inhibitor phenyl methane sulfonyl fluoride (PMSF) (1 mM), chymostatin (100 µM) and trypsin inhibitor (100 µg/ml), but not being inhibited by (4-amidinophenyl) methane sulfonyl chloride (APMSF) (50 µM) and N-[N-(L-3-trans-carboxyoxirane-2-carbonyl)-L-leucyl] agmatine (E-64) (2.5 µg/ml).

The present invention further provides a process for production of said serum calcium decreasing factor, characterized by isolating the serum calcium decreasing factor from the pancreas of a pig.

The present invention further provides a pharmaceutical composition comprising the serum calcium decreasing factor.

### BRIEF EXPLANATION OF DRAWINGS

Fig. 1 shows A profile of elution in Q-Sepharose fast flow chromatography. Only the peak III exhibits serum calcium decreasing activity.

In Fig. 2, A shows a profile of elution in a gel filtration chromatography by Superdex 75HR. A shows inhibition of calcium release (by PTH) in a Raisz's organ culture method, by some fractions corresponding to B. Only the main peak exhibits calcium release-inhibitory activity.

Fig. 3 shows a profile of elution in an ion exchange chromatography by Mono Q column. Only the main peak exhibits serum calcium decreasing activity.

Fig. 4 shows a profile of elution in a reverse phase chromatography by Wakosil 5C-18 column.

Fig. 5 is a graph showing that a serum calcium decreasing factor (open circles) and a serum calcium decreasing factor which has been treated with protease inhibitor PMSF (closed circles) lower serum calcium in a dose-dependent manner.

### BEST MODE FOR CARRYING OUT THE INVENTION

### PRODUCTION METHODS

The serum calcium decreasing factor of the present invention can be isolated and purified from porcine pancreas in the following manner. First, a pancreas is removed from a pig and, in a conventional manner, crushed and dehydrated with acetone to prepare acetone powder. The acetone powder is then extracted with an adequate buffer, for example, 0.1M Tris-hydrochloric acid containing 2%-NaCl (pH=8), to obtain an extract. To the extract is then added acetone to a concentration of 30%. After the precipitate is removed, acetone is again added to the supernatant to a concentration of 60% to obtain another precipitate.

The thus obtained 30 - 60% acetone fraction is dialyzed against an adequate buffer, for example, deionized water, to eliminate acetone, and aluminum sulfate is added to the solution to 45% saturation. The precipitate is then removed. To the supernatant is then added aluminum sulfate to 60% saturation and the formed precipitate is removed. The solution is dissolved in an adequate buffer, for example, 50 mM acetate buffer (pH 5.5) and is dialyzed against a buffer having the same composition as above to remove aluminum sulfate, to thereby obtain a crude extract of serum calcium decreasing factor.

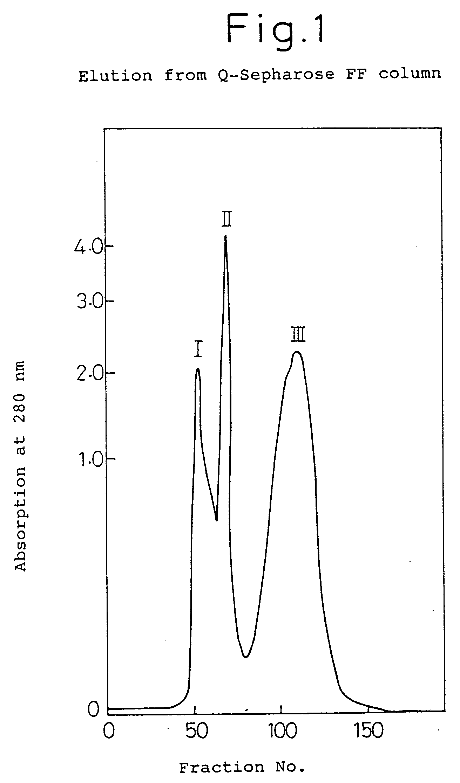

The serum calcium decreasing factor of the present invention is obtained from this extract in a process described in detail in Example 1. Briefly, said crude extract is applied to a Q-Sepharose Fast Flow column and 50 mM acetate buffer (pH 5.5) as well as 50 mM-acetate buffer containing 0.1M-NaCl are passed through the column, followed by eluting with acetate buffer containing 0.2M NaCl having the same composition as above to obtain three peaks as shown in Fig. 1. Only the material indicated by peak III exhibits the serum calcium decreasing effect in mice. The result is shown in Table 1 in Example 1.

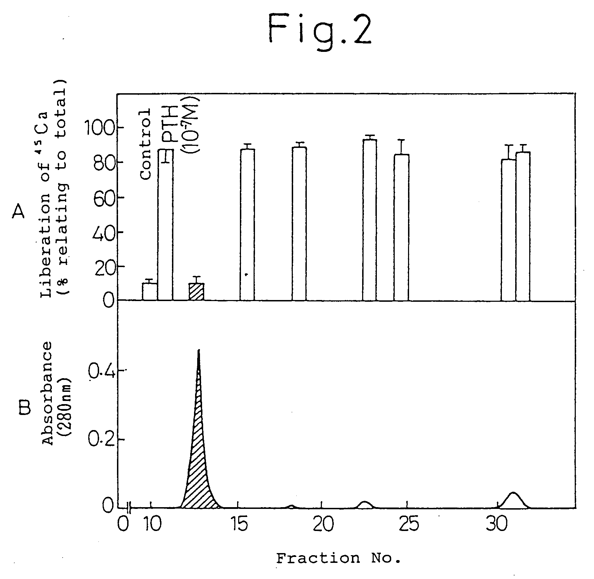

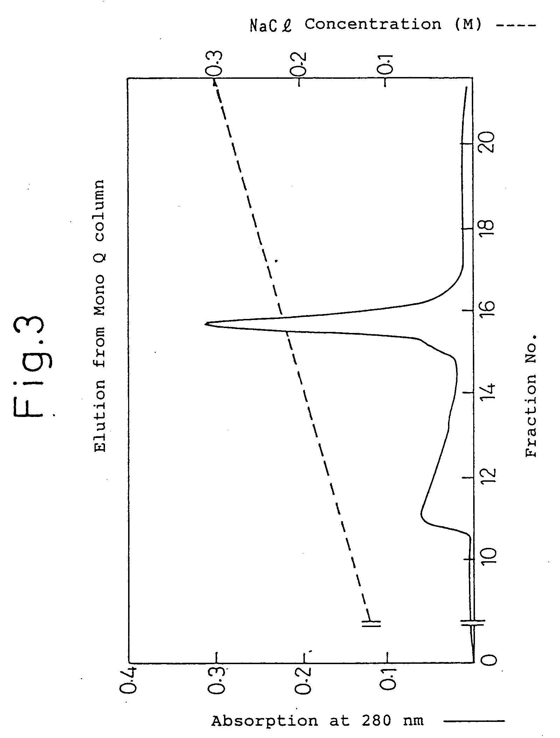

By gel filtration of said peak III through a Superdex 75HR column, one sharp main peak and three minor peaks as shown in Fig. 2B are observed. Only the main peak exhibits the serum calcium decreasing activity in vivo assay and the inhibition of parathyroid hormone (PTH)-derived calcium release in Raisz's organ culture method [Raisz L. G., J. Clin. Invest., 44, 103-116 (1965)]. These results are shown in Fig. 2A. The main peak obtained by said gel filtration chromatography is applied to ion exchange chromatography through a Mono Q HR column and is eluted with a NaCl concentration linear gradient in 50 mM sodium acetate buffer (pH 5.5). As a result, a main peak is eluted at 0.2M NaCl as shown in Fig. 3, which exhibits serum calcium decreasing effect in in vivo assay.

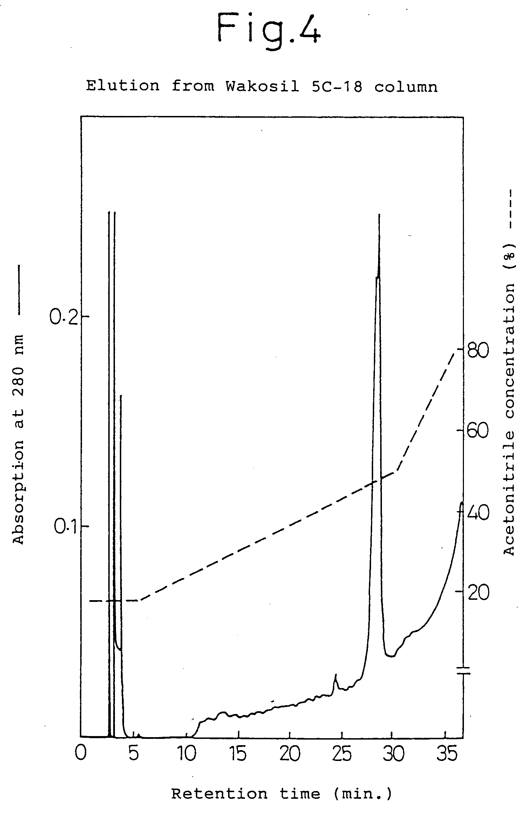

Next, the main peak obtained by gel-filtration chromatography is applied to reverse-phase chromatography and is eluted with acetonitrile concentration linear gradient in 0.1% trifluoroacetic acid (TFA). As a result, an elution profile as shown in Fig. 4 was obtained and it was confirmed that the serum calcium decreasing factor of the present invention was purified as a single protein by purification through a Mono Q HR column.

Although the serum calcium decreasing factor of the present invention was isolated and purified in the manner as above, it is clear that once it was isolated and purified and the properties thereof were analyzed as described below, the serum calcium decreasing factor of the present invention can be isolated and purified in any conventional methods used for isolation and purification of proteins.

It is also possible to prepare the serum calcium decreasing factor of the present invention by means of genetic engineering. For example, mRNA can be isolated from porcine pancreas in a conventional manner and a cDNA library can be prepared based on the mRNA in a conventional manner. A DNA probe for screening the cDNA library can be designed based on the amino acid sequence at or near the N-terminus of the serum calcium decreasing factor, which has been revealed by the present invention. Alternatively, after the serum calcium decreasing factor of the present invention is enzymatically digested or chemically cleaved and the amino acid sequence of the fragments be determined, the DNA probe can be designed based on the amino acid sequence.

The thus obtained cDNA that codes for a serum calcium decreasing factor is inserted into an adequate expression vector and a host is then transformed with the expression vector. By culturing the obtained transformant, the serum calcium decreasing factor of the present invention can be obtained. As the above host, conventional hosts such as procaryotic cells, e.g., E. coli, lower eukaryotic cells, e.g., yeast, higher eukaryotic cells, e.g., mammal cultured cells, and so on can be used.

### PROPERTIES OF SERUM CALCIUM DECREASING FACTOR

(1) Molecular Weight
   When a molecular weight of the present serum calcium decreasing factor is estimated by SDS-polyacrylamide gel electrophoresis, it shows a single molecular weight of about 26,500 to 28,000D.
(2) The present serum calcium decreasing factor shows an isoelectric point of about 4.5 as estimated by isoelectric focusing.
(3) When the N-terminal amino acid sequence of the purified serum calcium decreasing factor was determined by Edman degradation, it has the following amino acid sequence (SQ ID NO: 1) at the N-terminus or near the N-terminus: Val-Val-Gly-Gly-Gln-Asn-Ala-Ile-Pro-His-Ser-Trp-Pro-Trp-Gln-Ile-Arg-Leu-. This amino acid sequence has a homology to pig plasminogen, human-apolino-protein A, pig-elastase 2, bovine-chymotrypsinogen A, bovine-chymotrypsinogen B, human-plasma kallikrein (Pre) and human coagulation factor XI (Pre), but is not the same as these proteins and therefore, the present serum calcium decreasing factor is a novel protein.
(4) Effect of Inhibitors on Protease Activity
   The present serum calcium decreasing factor has protease activity and effects of protease inhibitors on the protease activity of the serum calcium decreasing factor are described in detail in Example 3 and Table 2.

### EFFECT

The present serum calcium decreasing factor has a serum calcium decreasing activity in vivo based on the inhibition of bone resorption, and therefore is expected to be useful as an active ingredient in pharmaceutical preparations for treatment and prophylaxis for various bone diseases such as osteoporosis, hyperparathyroidium, and hypercalcemia caused by malignant tumor. The present serum calcium decreasing factor can be formulated with a conventional pharmaceutical carrier, and can be administered by parenteral, for example intravenous, subcutaneous, intramuscular, or enteral or oral administration.

### Example 1. Purification of Serum calcium Decreasing factor from porcine pancrease

An acetone powder of a pig pancreas was prepared. The powder was extracted with a 0.1M Tris-HCl buffer containing 2% NaCl, and then an acetone precipitation fraction was obtained at an acetone concentration of 30 to 60%. This fraction was dialyzed against deionized water and then salted-out with ammonium sulfate to obtain a precipitation fraction at an ammonium sulfate 45 to 60% saturation.

The ammonium sulfate precipitation fraction was dissolved in a 50 mM acetate buffer (pH 5.5) and then dialyzed against a 50 mM acetate buffer to remove ammonium sulfate. Thus, a crude extract of serum calcium decreasing factor was obtained. The extract was subjected to ion-exchange chromatography using a Q-sepharose fast flow column. Most of the protein was passed with a 50 mM acetate buffer (pH 5.5). Then, stepwise elution was carried out with a 50 mM acetate buffer containing 0.1M NaCl and further stepwise elution was carried out with a 50 mM acetate buffer containing 0.2M NaCl. Thus, three peaks as shown in Fig. 1 were obtained.

Then, for these peaks, a serum calcium decreasing effect in mice was evaluated by the following method (in vivo measurement).

Male BALB/c mice of 4 to 6 weeks old were starved for 18 hours and 0.1M Tris-HCl buffer (pH 7.5) or 0.1M Tris-HCl buffer containing each of the three peaks dissolved therein was administered through a mouse tail vein at a dose of 100 µl per 20g of the body weight. After 4 hours, blood was drawn from the heart under ether anesthesia and serum was obtained. The serum calcium concentration was measured according to the orth-cresol phthalein complexion method.

The results are shown in Table 1 below.

**Table 1**

| Serum calcium decreasing activity of Q-sepharose fast flow column eluted peaks | | |
|---|---|---|
| Tested sample | Serum Ca concentration (mg/dl) | Significance of serum Ca concentration lowering |
| Control | 8.91±0.15 | |

| Peak I | | |
|---|---|---|
| 1.98 mg/kg weight | 8.90±0.20 | |

| Peak II | | |
|---|---|---|
| 1.73 mg/kg weight | 8.89±0.12 | |

| Peak III | | |
|---|---|---|
| 5.30 mg/kg weight | 8.25±0.23 | P<0.01^{(*)} |
| 0.53 mg/kg weight | 8.60±0.20 | P<0.05 |

| | | |
|---|---|---|
| (*) According to t test | | |

As is proved from the result of Table 1, the peak III exhibits serum calcium decreasing activity and a significant serum calcium decreasing activity is recognized even where the peak was diluted to a 1/10 concentration.

Then, the above-mentioned peak III was subjected to gel permeation chromatography. Namely, the peak III was introduced into a Superdex 75 HR 10/30 column in a 0.2M ammonium sulfate, eluted at 0.5 ml/mn, and fractionated to 1 ml fractions. The result is shown in Fig. 2B. Thus, one sharp main peak and three minor peaks were observed. The main peak, which corresponds to a molecular weight of about 22,000D, exhibited serum calcium decreasingeffect in in vivo measurement. Next, for several fractions including the fractions corresponding to the above-mentioned four peaks, inhibitory activity of the calcium release with parathyroid hormone (PTH) was evaluated using a Raisz's organ culture method.

ICR mice of 15 days pregnancy were subcutaneously administered with 10 to 20 µ Ci of ⁴⁵Ca, and the forearm bone of the fetus was cut on the next day and pre-cultured in an α-MEM medium. The medium was exchanged with α-MEM medium containing parathyroid hormone (PTH) and a test sample was cultured for 3 days. ⁴⁵Ca in bone is obtained by acid decalcification. The effect of calcium release by parathyroid hormone is expressed by a ratio of ⁴⁵Ca in the medium relating to a total amount of ⁴⁵Ca in the bone and medium. The results are shown in Fig. 2A. In Fig. 2, the location of a bar in Fig. 2A corresponds to the fraction number in Fig. 2B. As shown in this figure, PTH (10⁻⁷M) accelerated the release of calcium by about 80%, but the fraction corresponding to the main peak completely inhibited the acceleration of the calcium release. In contrast, neither the fractionS corresponding to the three minor peaks nor the fractions not corresponding to any of the main and minor peaks exhibited the inhibition effect.

The main peak was applied to polyacrylamide gel electrophoresis in the presence of sodium dodecylsulfate (SDS-PAGE) and was compared to a molecular weight standard (albumin 67KD, ovalbumin 43KD, carbonic anhydrase 30KD, trypsin inhibitor 20KD and α-lactalbumin 14KD) to reveal that the main band had a molecular weight of about 26,500D. The main peak obtained by said gel filtration chromatography was applied to ion exchange chromatography using a Mono Q HR 5/5 column. Using a buffer A, 50 mM sodium acetate (pH 5.5) and a buffer B, 50 mM sodium acetate containing 0.5M-NaCl, an elution was carried out with NaCl concentration linear gradient at a flow rate of 0.5 ml/min to fractionate to 1 ml fractions. The result is shown in Fig. 3. The main peak was obtained at a NaCl concentration of about 0.2M and this peak exhibited a serum calcium decreasingeffect in in vivo assay.

Next, the purity of the active main peak obtained from said ion exchange chromatography using Mono Q column was tested by reverse phase liquid chromatography. Elution was carried out by acetonitrile concentration linear gradient using solvent A [0.1% trifluoroacetic acid (TFA) in 20% acetonitrile] and solvent B [0.1% TFA in 80% acetonitrile] at a flow rate of 1 ml/minute. The elution was carried out by 100% A: 5 minutes; 0-50% B: 25 minutes; 50-100% B: 5 minutes; 100% B: 5 minutes. The result is shown in Fig. 4. A single peak was obtained at an acetonitrile concentration of about 50%, and it was confirmed that the present serum lowering factor was purified to a single protein.

Next, the molecular weight of the active peak obtained from the above-mentioned Mono Q ion exchange chromatography and the molecular weight of the peak obtained from Wakosil 5C-18 reverse phase liquid chromatography were estimated by non-reducing SDS-PAGE as above described. As a result, the molecular weight of the serum calcium decreasing factor was calculated as about 28,000D. Moreover, the isoelectric point (pI) of the fraction obtained from the above-mentioned Mono Q ion exchange chromatography was tested. As a result, the present serum calcium decreasing factor showed a single peak at a position of pI = 4.5, comparing with human carbolic anhydrase B: pI = 6.55, bovine carbolic anhydrase B: pI = 5.85, β-lactoglobulin A: pI = 5.2, soybean trypsin inhibitor: pI = 4.55.

### Example 2. Determination of Partial Amino Acid sequence

The main active fraction from the gel filtration chromatography using a Mono Q column and the fraction from the reverse phase liquid chromatography using Wakosil 5C-18 were separately investigated with usual degradation method for N-terminus amino acid sequence of the proteins. As a result, the above-mentioned two samples provide the same result, and it was assumed that a serum calcium decreasing factor of this invention has the following amino acid sequence (SEQ.ID NO: 1)at or around the N-terminus: Val-Val-Gly-Gly-Gln-Asn-Ala-Ile-Pro-His-Ser-Trp-Pro-Trp-Gln-Ile-Arg-Leu-.

It was then found that there was some homology of the protein with pig plasminogen, human-apoprotein A, pig elastase 2, bovine chymotrypsinogen A, bovine chymotrypsinogen B, human-plasmakallikrein (Pre) and human blood coagulation factor XI, when a test of homology was carried out for the 10-18th amino acid sequence, but the resulting sequence was not identical with all of the known active proteins. It was, accordingly, confirmed that the serum calcium decreasing factor of this invention was a novel protein.

### Example 3.

In steps of the purification, since it was observed that a fragment which was assumed to be a degraded fragment of the serum calcium decreasing factor protein, was generated, it is assumed that this serum calcium decreasing factor has proteolytic activity. An effect of various protease inhibitors against the serum calcium decreasing factor protein of this invention was tested as follows:
1 mg/ml azo-casein was used as a substrate, incubation was conducted at temperature of 37°C for 30 min in 0.25 ml, 1 ml of 5% trichloroacetic acid was added thereto, and then the supernatant from centrifugation of the reaction products was measured for OD at 340 nm. The resulting value was used as proteolytic activity. The effect of each protease inhibitor is shown based upon a percentage where proteolytic activity of a control sample without the inhibitor is 100%.

The protease inhibitors used include phenylmethansulfonyl fluoride (PMSF), which is a serin-protease inhibitor; (4-amidinophenyl)-methanesulfonyl fluoride (APMSF), which is a trypsin-type serin-protease inhibitor; leupetin, which is a trypsin-type serin-protease-thiolprotease inhibitor; chymostatin, which is a chymotrypsin-type serinprotease inhibitor; N-[N-3-trans-carboxyoxirane-2-carbonyl)-L-leucyl]agmatine (E-64), which is a thiolprotease inhibitor; pepstatin, which is an acid protease inhibitor; trypsin inhibitors which are inhibitors of trypsin, Xa-factor, plasmin and plasmakallikrein; and aprotinin, which is an inhibitor of trypsin and kallikrein.

As shown in table 2, activity of the present protein is inhibited by PMSF (1 mM), chymotrypsin (100 µM) and trypsin inhibitor (100 µg/ml), and is partially inhibited by leupeptin (100 µM), pepstatin (100 µM) and aprotinin (2 mg/ml), while activity of the protein is not inhibited by APMSF (50 µM) and E-64 (2.5 µg/m).

**Table 2**

| Effects of Various Proteases Inhibitors on Protease Activity of Serum Calcium Decreasing Factor | | |
|---|---|---|
| Inhibitor | Concentration of Inhibitor | Activity of Protease (per Activity of Control%) |
| Control (no addition) | 0 | 100 |
| PMSF | 1 mM | 8.8 |
| APMSF | 50 µM | 107.0 |
| Leupeptin | 100 µM | 86.8 |
| Chymostatin | 100 µM | 16.1 |
| E-64 | 2.5 µg/ml | 107.9 |
| Pepstatin | 100 µM | 67.9 |
| Trypsin inhibitor | 100 µg/ml | 4.4 |
| Aprotinin | 2 mg/ml | 78.7 |

### Example 4. Study on mechanism of serum calcium decreasing action

Since the serum calcium decreasing factor has protease activity, this factor possibly degrades parathyroid hormone (PTH). Therefore, the serum calcium decreasing factor and PTH were together incubated, and the reaction product was analysed by reverse phase chromatography. As a result, the PTH was degraded by the serum calcium decreasing factor to form a lower molecular weight product, but if PMS, a protease inhibitor, was present during this reaction, the degradation of PTH was inhibited.

Therefore, it was tested whether the serum calcium decreasing factor degradates PTH in an in vivo system wherein serum protein is present at a high concentration, or in Lewis bone culturing system.

It was tested whether the serum calcium decreasing factor inhibits calcium liberation by PTH or vitamin D in Raisz's organ culture method. As a result, although the serum calcium decreasing factor inhibited the effect of PTH at a low concentration of 10 mg/ml, it did not inhibit the effect of vitamin D.

Thus, to determine whether the inhibition to the effect of PTH is due to protease activity of serum calcium decreasing factor, it was tested whether the serum calcium decreasing factor, which has been treated with PMSF, that is, a protease inhibitor which is irreversibly binds to protease, inhibits calcium releasing activity of PTH and vitamin D. As a result, the serum calcium decreasing factor treated with the protease inhibitor PMSF inhibited the action of PTH, as the non-treated serum calcium decreasing factor did, but did not inhibit the action of vitamin D.

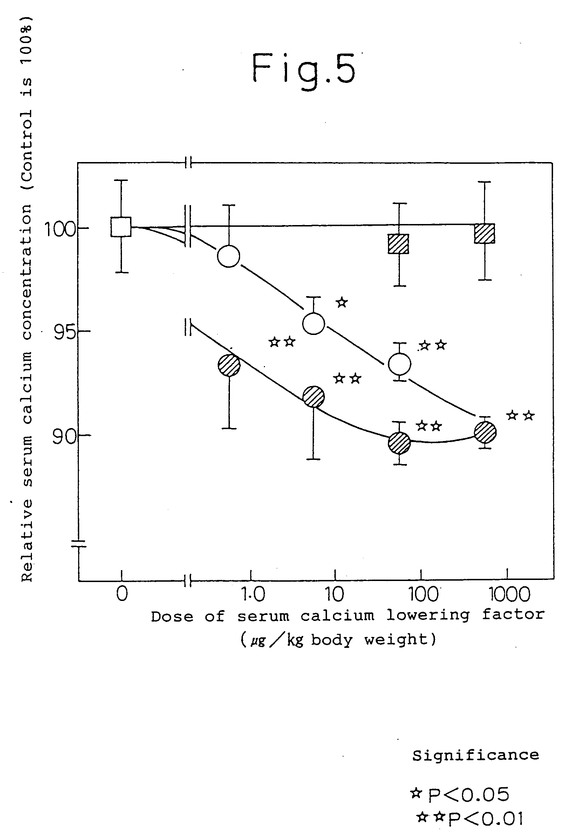

Next, lowering of serum calcium concentration by serum calcium decreasing factor in an in vivo assay on mice was tested using serum calcium decreasing factor treated with PMSF and non-treated serum calcium decreasing factor. The result is shown in Fig. 5. In the figure, open circles show the result of non-treated serum calcium decreasing factor, closed circles show the result of serum calcium decreasing factor treated with PMSF, and closed boxes show the result (control) wherein PMSF alone was administrated in an amount provided when serum calcium decreasing factor was administrated in an amount shown on the absissa.

Although SMSF did not lower serum calcium, PMSF-treated serum calcium decreasing factor also lowered serum calcium in a dose dependent manner as non-treated serum calcium decreasing factor did, and the curve shifted to the low concentration side. This result suggests that serum calcium decreasing factor, of which protease activity is inhibited, possibly still exhibits serum calcium decreasing activity.

### [Industrial Applicability]

Since the present serum calcium decreasing factor has in vivo serum calcium decreasing activity based on the inhibition of bone resorption, it promises to be useful as an active ingredient of pharmaceuticals for treatment and prophylaxis of various bone diseases such as osteoporosis, hyperparathyroidium, and hypercalcemia caused by malignant tumor.

SEQUENCE LISTING
SEQUENCE ID NO: 1
SEQUENCE LENGTH: 18
SEQUENCE TYPE: Amino acid
TOPOLOGY: Linear
MOLECULE TYPE: Peptide
SEQUENCE
Val Val Gly Gly Gln Asn Ala Ile Pro His Ser Trp Pro Trp
1               5                   10
Gln Ile Arg Leu
15

## Claims

1. A serum-calcium-decreasing factor of pig origin having the following properties:
(1) lowering a serum-calcium level in a dose-dependent manner in a mouse;
(2) having a molecular weight of about 26,500 to 28,000D, as estimated by SDS-polyacrylamide gel electrophoresis;
(3) having an isoelectric point of about 4.5 as estimated by isoelectric focusing;
(4) having the following amino acid sequence (SQ IN NO: 1) at the N-terminus or near to the N-terminus: Val-Val-Gly-Gly-Gln-Asn-Ala-Ile-Pro-His-Ser-Trp-Pro-Trp-Gln-Ile-Arg-Leu-;
(5) having protease activity, and being inhibited by protease inhibitor, phenylmethanesulfonyl fluoride (PMSF) (1 mM), chymostatin (100 /µM) and trypsin inhibitor (100 µg/ml), but not being inhibited by (4-amidinophenyl) methanesulfonyl fluoride (APMSF) (50 µM) and N-[N-(L-3-trans-carboxyoxirane-2-carbonyl)-L-leucyl] agmatine (E-64) (2.5 µg/ml):

2. A process for production of a serum calcium decreasing factor characterized by isolating the protein according to claim 1 from the pancreas of a pig.

3. A pharmaceutical composition comprising the serum calcium decreasing factor according to claim 1.
